# EUROPEAN PATENT SPECIFICATION

(11) **EP 2 801 320 B1**
(45) Date of publication and mention of the grant of the patent: **20.06.2018**
(21) Application number: 13166970.7
(22) Date of filing: 08.05.2013
(51) Int. Cl.: A61B 6/12, A61B 6/00, A61B 6/04, A61B 17/56

(54) **C-arm adjustment**
C-Bogeneinstellung
Réglage de bras en C

(43) Date of publication of application: 12.11.2014
(73) Proprietor: Stryker European Holdings I, LLC, Kalamazoo, MI 49002 (US)
(72) Inventor: Blau, Arno, 79194 Gundelfingen (DE); Simon, Bernd, 24107 Kiel (DE); Metz, Lars, 24148 Kiel (DE)
(74) Representative: Schmitz, Alexander

(56) References cited:
- WO-A1-00/56215
- WO-A1-2004/021910
- WO-A1-2012/068679
- WO-A1-2012/100825
- US-A1- 2013 094 742

## Description

### FIELD OF THE INVENTION

The invention relates to the field of computer assisted surgery. In particular, the invention relates to a method and a device for assisting a C-arm adjustment. The method may be implemented as a computer program executable on a processing unit of the device.

### BACKGROUND OF THE INVENTION

In every surgery where intraoperative imaging is used it is a challenge to correctly position the C-arm of an imaging device. This is at most an iterative positioning procedure based on several X-ray images. A significant radiation exposure is thus produced only in order to find the best C-arm position. The amount of radiation may be highly dependent on the know-how and skill of the C-arm technician (if available at all). In order to support this iterative procedure, some C-arm models offer a laser beam feature that addresses this problem, but still it is an iterative procedure.

Some systems may provide virtual information about current position of an implant related to a bone. Also it may be able to provide information on how to adjust the current implant or instrument position to optimise the current procedural steps. However, the availability of those information is highly dependent on the visibility of necessary structures in the image, like a reference body, a certain anatomical structure, an aiming device, a drilling sleeve or the like.

Whenever at least one of the needed structures is not visible, the virtual information cannot be provided. The C-arm user should be aware of this and should correct the C-arm position accordingly if needed. Also this is most likely an iterative procedure until a sufficient C-arm position is found.

DE 103 35 656 A1 describes a method and a device for controlling movements of components of an X-ray diagnostic system. Here, a plurality of X-ray images from different imaging directions are generated and points at corresponding positions are manually marked in each of the images. Based on these marked images and based on an input representing an intended imaging direction, a movement of the X-ray imaging system is calculated, so that the system may provide an image based on the intended imaging direction.

Document WO 2012/068679 A1 (CLARON TECHNOLOGY INC, 31 May 2012) may be considered to discloses a method comprising the steps of: receiving a 2D projection image of an anatomical structure from a CT device, the 2D projection image including a visualization of a reference body having a structure allowing a determination of an orientation of the reference body based on the 2D projection image, determining the orientation of the reference body based on the 2D projection image, determining a specific orientation relative to the anatomical structure based on the orientation of the reference body, with the reference body having a predetermined relation to the anatomical structure; and also to disclose the corresponding device.

### SUMMARY OF THE INVENTION

It may be seen as an object of the invention to provide a method and a device for more effeciently assisting an adjustment of a C-arm. It would be of high benefit to reduce the amount of radiation to which a patient is exposed, and to have a more effectient way to directly change the position of the C-arm for either optimise visibility of structures in current images or move to a next workflow-based position.

This is achieved by the subject-matter of each of the independent claims. Further embodiments are described in the respective dependent claims.

In general, a method for assisting in C-arm adjustment comprises the steps of receiving a 2D projection image of an anatomical structure from a C-arm based imaging device, the 2D projection image including a visualization of a reference body having a structure allowing a determination of a 3D orientation of the reference body relative to the anatomical structure based on the 2D projection image, determining the 3D orientation of the reference body, determining an imaging direction based on the 3D orientation of the reference body, determining a body plane based on the 2D projection image, and determining an angle between the viewing direction and the body plane.

According to an embodiment, a single 2D projection image is received as a basis for, finally, the determination of the angle between the viewing direction and the body plane.

The body plane may be a plane which is at least parallel to a sagittal plane, a frontal plane or a transversal plane. Assuming that a person is standing, "transversal plane" would mean a horizontal plane dividing the body of the person into an upper portion and a lower portion. "Frontal plane" means a plane which divides the body of a person into a front part and a back part and "sagittal plane" means a plane which divides the body of a person into a right part and a left part. These denotations also count for each part of a body, like an arm or a leg or the head, independent from the current orientation relative to the rest of the body. Even if for example the head is turned to one side, the sagittal plane of the head divides the head into a right part including the right eye and ear, and a left part including the left eye and ear, although this plane would be at least inclined relative to the sagittal plane of the rest of the body.

According to an embodiment, the body plane is determined based on at least one anatomical feature of the anatomical structure, in particular on at least one bone feature.

As used herein, the term "feature of a bone" refers to anything at a bone which may be suitable for determining a geometrical aspect, i.e. a point, a line, an arc, a center point, an axis, a cylinder surface, a ball surface, or the like, wherein such geometrical aspects are in particular suitable for a determination of a body plane. For example, a geometrical aspect of a femur may be the outer surface of the femur head, an axis defined by the neck between shaft and femur head, a longitudinal axis of the femur shaft, a most distal point on the bone surface, a line defined by the center points of the condyles, or a line defined by the most posterior points at the condyles. It will be understood that the other bones provide other and/or comparable suitable geometrical aspects.

In case an implant is already implanted into or at a bone, a feature of the implant may also be determined instead of a feature of the bone so that the feature of the implant may represent a feature on the basis of which a body plane may be determined. Consequently, the 2D projection image may include a visualization of an implant, with a feature of the implant being determined and being utilized for determining a body plane.

According to an embodiment, the determination of the body plane may be further based on 3D data received from a data base, the 3D data representing a corresponding anatomical feature. In such 3D data the body planes may be already determined and may thus be transferred to an anatomical structure visible in the 2D image.

According to a further embodiment, the body plane may be determined based on the 3D orientation of the reference body, with the reference body being adapted to be positioned in a fixed and predetermined manner relative to the anatomical structure. The reference body may define at least one axis and a point defining a plane.

As used herein, "in a fixed and predetermined manner" encompasses a direct or an indirect connection of a reference body to an anatomical structure. The reference body may be directly attached to an anatomical structure, i.e. may be in contact with an outer surface of a body part of interest. The reference body may be indirectly coupled to an anatomical structure, for example via an aiming device.

On the other hand, the reference body may be at least a part of an implant. In other words, an implant which is adapted to be fixed at or in a bone may comprise elements which can be identified in an image of the bone or at least a section of the bone so that a vector and/or plane may be determined based on the identified elements. For example, the elements may define points so that two elements may define a line or an axis, or the elements may define a contour so that a center axis may be determined.

According to another example, the reference body may be integrated into an aiming device for supporting an insertion of a locking screw through a bore in a leading end of a bone nail. Therefore, the aiming device may be adapted to be coupled to a trailing end of the bone nail and may extend outside the body of a patient as far as the bone nail extends inside the bone so that at least a portion of the aiming device can be visible in an image of the section of the bone including the leading end of the bone nail.

According to an embodiment, the method may further comprise the steps of receiving an input representing an intended viewing direction and providing information for an adjustment of the C-arm based imaging device to achieve the intended viewing direction for a next image. It is noted that the intended viewing direction may be a direction predetermined by a specific workflow of a surgical treatment procedure.

For example based on a 2D image generated with a viewing direction within a frontal plane, a lateral deviation of a visualized feature of an implant relative to an expected visualization of the feature of the implant may be determined. Based on the deviation, a lateral bending of the implant within the frontal plane may be determined.

According to an embodiment, the method does not comprise any step of positioning a reference body, in so far as it constitutes a treatment of a human or animal body by surgery.

According to a further aspect, a device for assisting in C-arm adjustment comprises a reference body having a structure allowing a determination of a 3D orientation of the reference body relative to an anatomical structure based on a 2D projection image, a receiving unit for receiving a 2D projection image of an anatomical structure together with a reference body from a C-arm based imaging device, a processing unit configured for determining the 3D orientation of the reference body based on the 2D image, wherein the processing unit is further configured for determining an imaging direction based on the 3D orientation of the reference body, for determining a body plane based on the 2D projection image, and for determining an angle between the viewing direction and the body plane.

It is noted, that the processing unit may be realized by only one processor performing all the steps of the method, or by a group or plurality of processors, for example a system processor for processing the image data, a separate processor specialized on a determination of geometrical aspects, and a further processor for controlling a monitor for visualizing results.

According to an embodiment, the processing unit of the device may be further adapted to automatically identify a reference body in a projection image and to determine a 3D orientation of the reference body.

According to another embodiment, the device may further comprise an input unit for receiving an input representing an intended viewing direction. The input unit may be for example a computer keyboard, a computer mouse or a touch screen, so that the input unit may be adapted for manually identifying geometrical aspects of an anatomical structure like a bone in an image. Otherwise, the input unit may be an electronic interface for receiving data sets representing a workflow of a procedure including information on viewing directions.

The device may further comprise an output unit for providing data representing information for an adjustment of the C-arm based imaging device so as to achieve the intended viewing direction. According to an embodiment, the output unit may be a monitor for visualizing the information for an adjustment of the C-arm based imaging device. Based on this information, a C-arm user may manually adjust the imaging or viewing direction of the C-arm.

According to a further embodiment, the device may further comprise an imaging unit for providing 2D projection image data of at least a section of an anatomical structure. The imaging unit may be a C-arm based imaging unit capable of generating images from different directions. Accordingly, the imaging unit of the device may be adapted to also provide 3D image data of at least a section of the anatomical structure.

According to a further aspect, a computer software is provided including sets of instructions which when executed on an appropriate device, causes the device to perform the steps of the method as described above.

According to an embodiment, the computer software may be configured to automatically adjust the imaging direction of the C-arm based imaging device based on the data provided by the output device.

A corresponding computer program is preferably loaded into a work memory of a data processor. The data processor or processing unit is thus equipped to carry out the method. Further, the invention relates to a computer-readable medium such as a CD-ROM at which the computer program may be stored. However, the computer program may also be presented over a network like the World Wide Web and can be downloaded into the working memory of the data processor from such a network.

It has to be noted that embodiments are described with reference to different subject-matters. In particular, some embodiments are described with reference to method type claims (computer program) whereas other embodiments are described with reference to apparatus type claims (system). However, a person skilled in the art will gather from the above and the following description that unless other notified in addition to any combination of features belonging to one type of subject-matter also any combination between features relating to different subject-matters is considered to be disclosed with this application.

The aspects defined above and further aspects, features and advantages of the present invention can also be derived from the examples of the embodiments to be described hereinafter and are explained with reference to examples of embodiments also shown in the figures, but to which the invention is not limited.

### BRIEF DESCRIPTION OF THE DRAWINGS

Fig. 1 shows a flow chart of steps of an embodiment of a method.
Fig. 2 shows a flow chart of steps of another embodiment of the method.
Fig. 3 shows a schematical illustration of a system.
Fig. 4 shows a visualization of a distal section of a femur generated in a substantially lateral and inclined direction.
Fig. 5 shows an exemplary illustration of a femur in an anterior to posterior direction.
Fig. 6 shows an exemplary illustration of a femur in a proximal to distal direction.
Fig. 7 illustrates a deviation of a visualized feature of an implant relative to an expected visualization of the feature of the implant.

Throughout the drawings, the same reference numerals and characters, unless otherwise stated, are used to denote like features, elements, components, or portions of the illustrated embodiments. Moreover, while the present disclosure will now be described in detail with reference to the figures, it is done so in connection with the illustrative embodiments and is not limited by the particular embodiments illustrated in the figures.

### DETAILED DESCRIPTION OF THE EMBODIMENTS

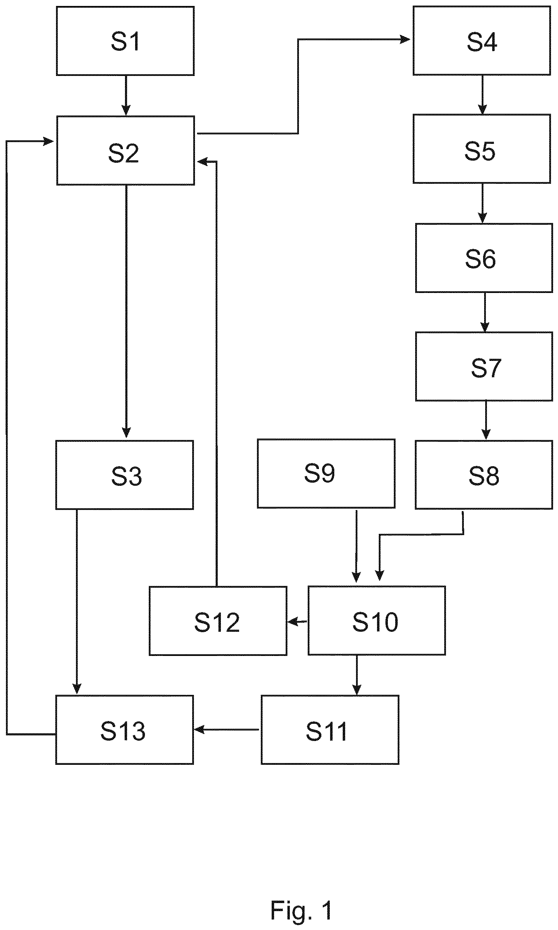

The flow-chart in Fig. 1 illustrates the principle of the steps performed in accordance with an embodiment. It will be understood that the steps described, are major steps, wherein these major steps might be differentiated or divided into several sub-steps. Furthermore, there might be also sub-steps between these major steps.

In step S1, a reference body is positioned relative to an anatomical structure. As the reference body comprises a structure allowing a determination of the 3D orientation of the reference body based on a 2D projection image, the reference body should be positioned so that the reference body will be imaged together with the anatomical structure of interest, i.e. the reference body will appear in the image of the anatomical structure. An appropriate position of the reference body is beside or above, i.e. nearby the anatomical structure. It is not necessary that the reference body is inside the anatomical structure.

In step S2, the anatomical structure together with the reference body is imaged by means of an imaging device like a C-arm based X-ray imaging device. The imaging device provides image data which may be transmitted to an output unit like a monitor as well as to a processing unit so as to further process the image data.

In step S3, the image data provided by the imaging device are visualized. For example, the X-ray image is shown on a monitor.

In step S4, the image data are received by a receiving unit. As used herein, the term "receiving an image" basically refers to the fact that at least one image is necessary to perform the subsequent steps. That is, the term "receiving an image" may encompass also a loading of an image from a data memory into a processing unit. There is no need to generate a new image to receive an image. Accordingly, each image received in step S4, may be any image suitable to identify a feature of a bone as an anatomical structure, i.e. may be a 2D image of at least a section of the bone, wherein the 2D image should additionally show the reference body.

It will be understood that a single image may be sufficient to determine a feature of a bone like an axis, suitable to determine a body plane.

In step S5, a 3D position and orientation of the reference body is determined. The position of the reference body may be determined in form of distances of specific points of the reference body in relation to the anatomical structure of interest. The distances may be measured horizontally and vertically between a predetermined and fixed point at the anatomical structure and the reference body. The orientation may be determined based on distances of at least two points of the reference body and the predetermined point at the anatomical structure. It is noted that the reference body may be directly or indirectly coupled to the anatomical structure to provide a predetermined relation between the reference body and the anatomical structure.

In step S6, an imaging direction is determined based on the 3D orientation of the reference body. The reference body has a unique and characteristic projection in any arbitrary projection direction, when being put into the radiation beam between a radiation source and a radiation sensor. The unique projection can be achieved, for example, when designing the reference body in a particular outer shape which is visible when being imaged, or by providing a particular distribution of fiducial markers. The reference body may be formed by a plurality of fiducial markers like radiopaque and spherical elements, which may be provided at or in a transparent body or at or in a tool like an aiming device. The fiducial markers may also be provided at or in an implant. Based on the unique projection, an actual orientation of the reference body can be determined.

In step S7, a body plane is determined. As indicated above, a body plane may be a sagittal plane, a transversal plane or a frontal plane. Each of these planes has a specific orientation relative to an anatomical structure. Furthermore, each of these planes has a specific orientation relative to a specific body feature, in particular to a specific bone feature, as bone provides a rigid structure. A body plane may therefore be determined based on a bone feature visible in an X-ray image.

In step S8, an angle between the imaging direction and the body plane is determined. In a case in which more than one body plane is determined, more than one angle may be determined. Knowing the relation between the reference body and the anatomical structure as well as the relation between the reference body and the imaging direction allows a determination of an angle between the anatomical structure and the imaging direction.

In step S9, which is a separate step, a user may provide an input representing an intended imaging direction. For example, it may be of interest to generate an image in a direction parallel to a frontal plane.

It is noted that it is not necessary to receive an input from a user. An intended imaging direction may also be provided as a next workflow based position.

Based on the input, information may be provided in step S10 as to how the imaging direction should be changed or adjusted starting from the current adjustment (position and orientation) of the imaging device. The information may be provided as computer data representing new coordinates, i.e. detailed adjustment information for the C-arm of an imaging device.

According to an embodiment, the information is provided in step S11 as an output for the user. The output may be visible for example on a monitor or as an indication directly at the movable parts of the C-arm, but may also be an acoustic signal, for example providing instructions by an artificial voice as to how the C-arm should be adjusted to achieve the intended imaging direction. Based on the provided output, the user may manually adjust the C-arm to the intended position and orientation in step S13.

According to another embodiment, the information is further processed in step S12 by the processing unit so as to provide sets of computer instructions for automatically adjusting the C-arm. It will be understood that, in this case, the C-arm should be motorized.

As indicated by the arrows from S12 as well as from S13 back to S2, a next image may be generated with the automatically or manually adjusted imaging direction of the imaging device.

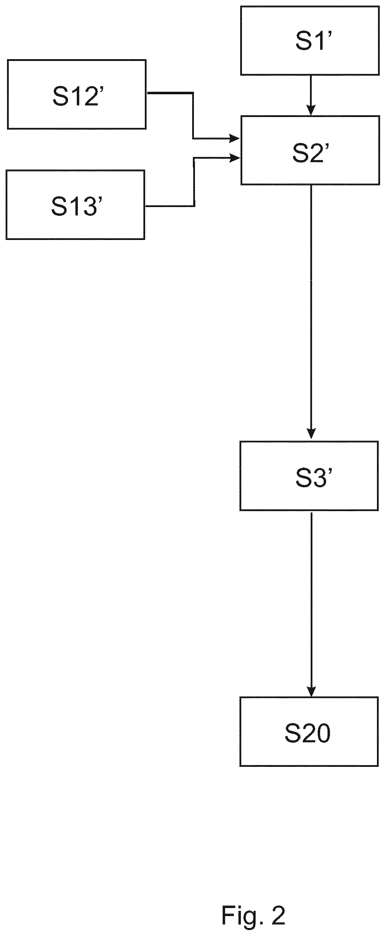

The flow-chart in Fig. 2 illustrates the principle of the steps performed in accordance with a further embodiment. Also here, it will be understood that the steps described, are major steps, wherein these major steps might be differentiated or divided into several sub-steps. Furthermore, there might be also sub-steps between these major steps.

With reference to Fig. 2, steps are described to achieve a determination of a deviation between an actually imaged feature of an implant and an expected feature of the implant.

For example, an intramedullary nail may have a specific shape and dimension. Accordingly, the nail may have an appearance in an X-ray image, which may be expected. However, it may occur that the nail will be deformed, for example bent, during an insertion of the nail into an intramedullary channel of a long bone. In those cases the appearance in an X-ray image will differ from the expected appearance.

The steps S1', S2', S3', S12' and S13' in Fig. 2 are comparable with the respective steps S1, S2, S3, S12 and S13 in Fig. 1, with the limiting condition that in the embodiment of Fig. 2 the imaging direction is automatically (step S12') or manually (step S13') adjusted to a specific direction, for example being within the frontal plane of the anatomical structure of interest. That is, the reference body may be positioned in step S1' so as to be substantially beside the anatomical structure, for example beside a leg. Further, the image may be generated with an imaging direction being within the frontal plane and being additionally inclined to the transcersal plane in step S2'. The image generated from this specific imaging direction is visualized in step S3'.

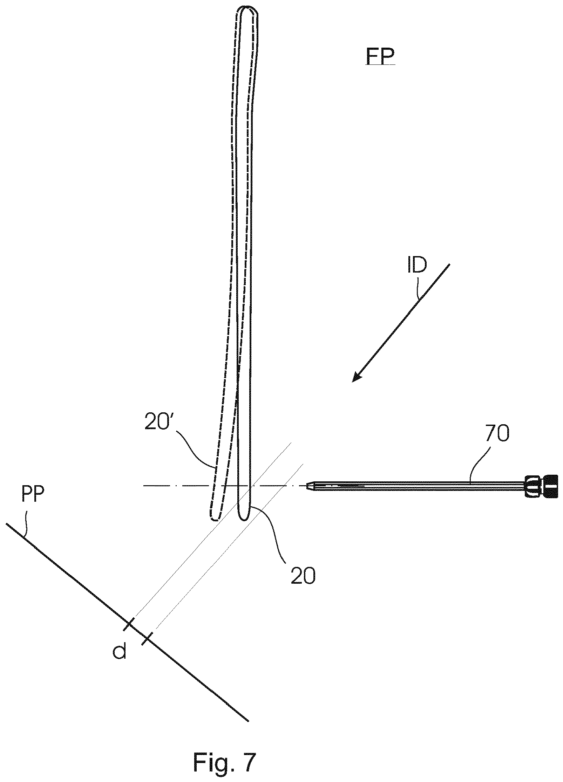

Based on such an image, a bending of an intramedullary nail in a lateral direction may be detected and an amount of the bending may be determined. This is described in more detail with reference to Fig. 7.

It will be understood that the embodiment of Fig. 2 is only one possible example and that based on other imaging directions also other deformations and/or deviation of any implant may be determined.

In general, a method for determining a deviation between an expected and an actual position of an implant inserted into an anatomical structure, may comprise the steps of imaging an anatomical structure of interest from a specific imaging direction, the image including a visualization of a reference body and of an implant, the reference body being positioned in a predetermined relation to the anatomical structure as well as to an implant, determining a position of a feature of the implant as expected due to the shape and size of the implant, determining an actual position of the feature of the implant based on the image, and determining a deviation between the two determined positions.

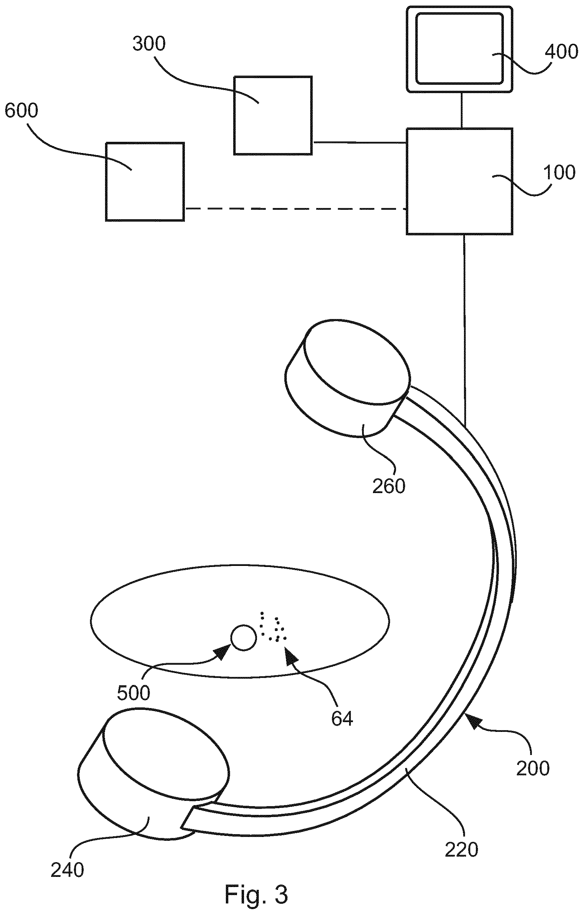

Fig. 3 shows an exemplary embodiment of a device. Substantially, necessary for performing the steps of the method, a processing unit 100 is part of the device.

An exemplary imaging device 200 includes an X-ray source 240, and an X-ray detector 260, wherein these two units are mounted on a C-arm 220.

Furthermore, the system in figure 3 includes an input unit 300, by means of which for example an intended imaging direction may be manually entered. Also shown is a connection (as dotted line) to a database 600, located for example in a network. The database 600 may comprise information regarding anatomical structures, for example from 3D scans of different anatomical structures, so that the imaged anatomical structure may be compared with this information so as to determine a body plane of the imaged anatomical structure.

Finally, there is shown an anatomical structure of interest 500 as well as a reference body 64 formed by a plurality of radiopaque spheres. Within said anatomical structure, for example a bone of a patient may be located which may be subject to the described methods.

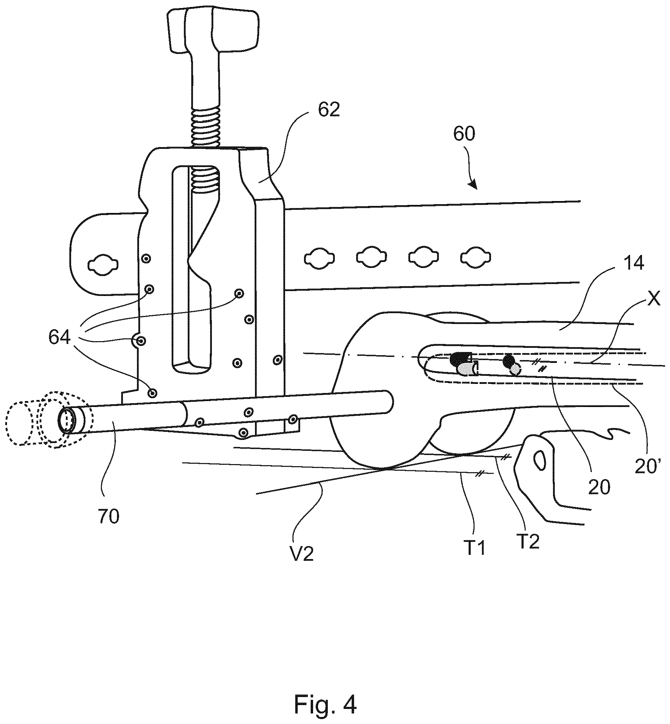

Figure 4 shows a schematic image which would be appropriate to determine most posterior points of the condyles as a feature of a femur. The image is generated from a substantially lateral direction but also inclined in a proximal to distal direction so that both condyles at the distal section 14 of the femur can be identified in one image. Additionally shown in the image of figure 4, is an aiming device 60 including an adjusting element 62 for adjusting a height of a sleeve for inserting a locking screw through a bore in the leading end of a bone nail 20, with the sleeve 70 being adapted to facilitate an insertion of a locking screw.

It is noted that the image would be generated exactly from a lateral direction, i.e. within the frontal plane of the femur, when a projection of the axis of the sleeve 70 would be parallel to the projection of the longitudinal axis X of the femur.

Further shown in figure 4 is a plurality of elements 64 forming an example of a reference body. Each of these elements 64 is a small radiopaque sphere so that each element is shown as a point in an X-ray projection image. Due to a specific 3D distribution of the elements 64 at or within the adjusting device 62, a projection of the elements onto an imaging plane will lead to a unique 2D distribution so that an actual 3D orientation of the reference body can be determined based on the projected 2D distribution. Knowing the 3D orientation of the reference body allows the determination of an actual direction of for example a tangent line T1, T2 at the condyles, and allows the determination of the actual imaging direction.

Based on the tangent lines T1, T2 and/or the axis X of the femur, at least one body plane, for example the transversal plane may be determined.

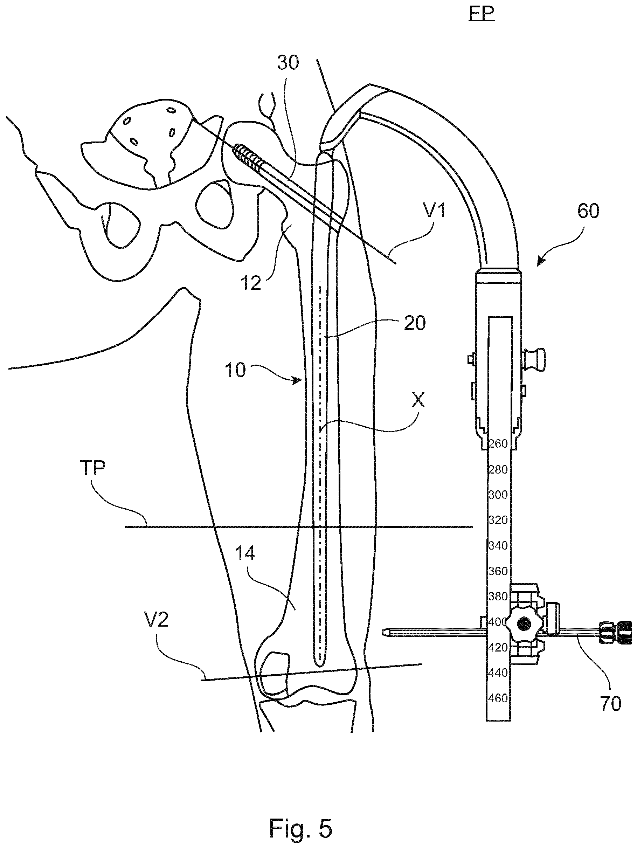

Figure 5 is an image generated in an anterior to posterior direction, i.e. viewing onto the frontal plane FP of the leg. Figure 5 shows a situation in which a bone nail 20 is already inserted into a medullary channel of the femur 10 in a longitudinal direction of the femur having a proximal section 12 into which a locking screw 30 is inserted, through a bore in the bone nail and into the head of the femur. Fixedly attached to the bone nail 20 is an aiming device 60 with a sleeve 70 for an insertion of a further locking screw to be inserted in the distal section 14 of the femur.

Furthermore, a transversal plane TP is shown being perpendicular to the longitudinal axis of the bone (perpendicular to the drawing sheet). For determining the frontal and/or the transversal plane, a first vector V1 at the condyles and a second vector V2 through the femur neck may be firstly determined, each of the two vectors representing a direction of a feature of the femur.

It is noted that the vectors V1 and V2 are not shown as arrows, since it is irrelevant in which direction the vectors point, along the shown lines, respectively.

As a first approximation it can be assumed that the longitudinal axis X of the bone nail 20 is identical with the longitudinal axis of the shaft of the femur 10. A transversal plane TP extending perpendicular to the longitudinal axis of the bone nail 20 will be identical to a plane extending perpendicular to the shaft of the femur, as long as the longitudinal axes are congruent or at least parallel to each other. In case there exists a deviation of the longitudinal axis of the bone nail from the longitudinal axis of the femur shaft, the angle between the longitudinal axes would be so small that a resulting error in the determination of the plane can be neglected. Therefore, the longitudinal axis X of the bone nail 20 which may be easier to determine, may be utilized to determine the transversal plane TP.

Having a locking screw 30 already inserted into the neck and head of the femur 10, provides the possibility to utilize the axis of the locking screw as a feature to determine a body plane.

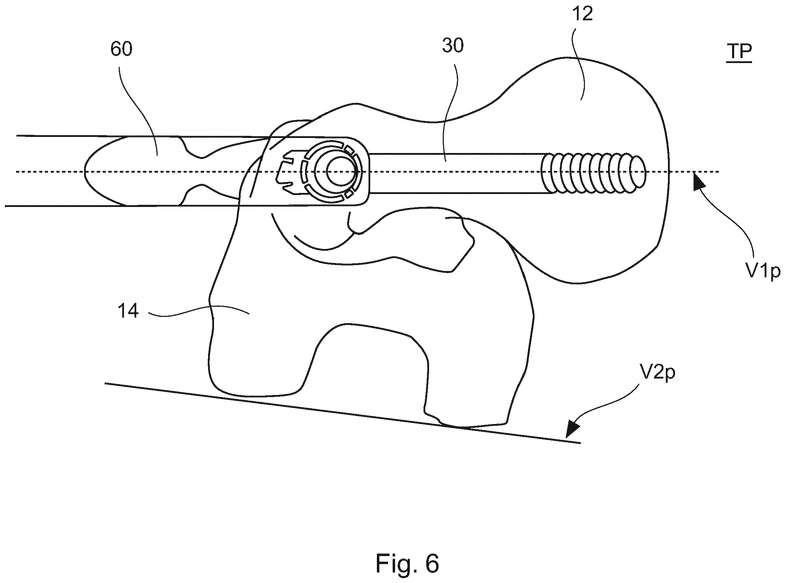

In figure 6, a plan view onto the transversal plane TP (identical with the drawing sheet) is shown together with features of the femur, i.e. the head of the femur at the proximal section 12 as well as the condyles at the distal section 14 of the femur, as would be seen when viewed in a proximal to distal direction of the femur. Figure 6 further shows a locking screw 30 extending from the bone nail into the head of the femur and an aiming device 60 which is fixedly connected to the bone nail and extends outside of the bone, i.e. the leg. It is noted that figure 6 is a constructed image for explanation purposes. Therefore, the soft tissue surrounding the femur is omitted in figure 6.

As can be seen in figure 6, the most posterior points at the condyles are used to determine a tangent line. The tangent line at the condyles may serve to determine a body plane of the femur. At the proximal section 12 of the femur, the axis of the locking screw 30 may be a basis for determining a body plane.

Fig. 7 illustrates the possibility to determine a deviation between an expected and an actual position of a feature of an implant. In bold line, an implant 20, i.e. an intramedullary nail, is shown, the implant 20 having a shape before insertion, i.e. without any forces acting on the implant. In dashed line, the implant 20' is shown as it might be actually shaped when being implanted. As the path through an intramedullary channel usually has a slightly different shape, an intramedullary nail which is to be inserted into the intramedullary channel, may be deformed due to forces acting from the wall of the intramedullary channel onto the nail.

Assuming that the plane of the drawing sheet of figure 7 represents a frontal plane FP, a lateral bending of the implant is shown from an original shape denoted with reference number 20, to a deformed shape denoted with reference number 20'. It will be understood that a bending to the front (anterior) or to the back (posterior) would be visible when the plane of the drawing sheet of figure 7 would be a sagittal plane. That is, a deviation between an expected and an actual shape can be easily determined perpendicular to the imaging direction.

As another example, both an expected and an actual shape of the distal end of an intramedullary nail is depicted in figure 4, as imaged from an inclined more or less lateral direction. In such a view it is difficult to differentiate between a lateral and a downward (or upward) deformation of the nail. Therefore, it may be of interest to adjust the C-arm of the imaging device in such a way that an image can be generated exact from lateral, i.e. in a frontal plane, so as to be able to clearly see a downward or upward bending. The determination of such a bending facilitates an adjustment of a height of the sleeve 70 which is adapted to guide a locking screw into a transverse bore in the nail.

In an image generated from an imaging direction lateral and perpendicular to the nail axis, i.e. in a direction of the axis of the sleeve 70, as shown in figure 7, a lateral bending is hardly visible.

In a specific case, namely with an imaging direction ID, as shown in figure 7, in the frontal plane and inclined, for example with an angle of 30 to 60 degree, preferably with an angle of 45 degree, to the sagittal plane (or transversal plane), it is possible to determine both a lateral and a downward (upward) deformation based on one image. The downward (upward) deformation is directly visible in the projection image. The lateral deformation can be indirectly determined due to the fact that the projected length of the nail differs depending from the amount of lateral deformation. In the example of figure 7, a length d may be measured in the projection plane PP between the projection of the tip of the nail as expected and the projection of the actual tip of the nail.

Knowing the imaging direction and the dimensions of the implant allows a determination of a deformation of the implant within the plane including the imaging direction.

While embodiments has been illustrated and described in detail in the drawings and aforegoing description, such illustrations and descriptions are to be considered illustrative or exemplary and not restrictive, the invention is not limited to the disclosed embodiments.

Other variations to the disclosed embodiments can be understood and effected by those skilled in the art in practising the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims.

The mere fact that certain measures are recited and mutually different dependent claims does not indicate that a combination of these measures can not be used to advantage. A computer program may be stored/distributed on a suitable medium such as an optical storage medium or a solid-state medium supplied together with or as a part of another hardware, but may also be distributed in other forms, such as via the Internet or other wired or wireless telecommunication systems. Any reference signs in the claims should not be construed as limiting the scope.

### LIST OF REFERENCE SIGNS

- 10: bone/femur
- 12: proximal section of bone/femur
- 14: distal section of bone/femur
- 20, 20': implant/bone nail
- 30: locking screw
- 60: aiming device
- 62: adjusting device
- 64: reference body
- 70: sleeve
- 100: processing means
- 200: imaging device
- 220: C-arm
- 240: X-ray source
- 260: X-ray detector
- 300: input device
- 400: monitor
- 500: region of interest
- 600: database
- d: length
- FP: frontal plane
- ID: imaging direction
- PP: projection plane
- TP: transversal plane
- T1, T2: tangent
- V1: first vector
- V2: second vector
- X: longitudinal axis

## Claims

1. A method for assisting in C-arm adjustment of an intended viewing direction, the method comprising the steps of:
receiving a 2D projection image of an anatomical structure from a C-arm based imaging device (200), the 2D projection image including a visualization of a reference body (64) having a structure allowing a determination of a 3D orientation of the reference body based on the 2D projection image,
determining the 3D orientation of the reference body based on the 2D projection image,
determining a plane having a specific orientation relative to the anatomical structure based on the 3D orientation of the reference body, with the reference body having a predetermined relation to the anatomical structure,
determining an angle between the viewing direction and said anatomical structure.

2. The method of claim 1, wherein said plane having the specific orientation relative to the anatomical structure is a plane which is at least parallel to a plane out of the group consisting of sagittal plane, frontal plane (FP) and transversal plane (TP).

3. The method of claim 1, wherein said plane having the specific orientation relative to the anatomical structure is further determined based on at least one anatomical feature of the anatomical structure, in particular on at least one bone feature.

4. The method of claim 3, wherein determining said plane having the specific orientation relative to the anatomical structure is further based on 3D data received from a data base (600), the 3D data representing a corresponding anatomical feature.

5. The method of claim 1, wherein the 2D projection image further includes a visualization of an implant (20) and wherein said plane having the specific orientation relative to the anatomical structure is determined based on a feature of the implant.

6. The method of claim 5, the method further comprising the step of determining a deviation of the visualized feature of the implant and an expected visualization of the feature of the implant.

7. The method of claim 1, further comprising the steps of receiving an input representing an intended viewing direction and providing information for an adjustment of the C-arm based imaging device to achieve the intended viewing direction.

8. A device for assisting in C-arm adjustment, the device comprising
a reference body (64) having a structure allowing a determination of a 3D orientation of the reference body based on a 2D projection image,
a receiving unit for receiving a 2D projection image of an anatomical structure from a C-arm based imaging device,
a processing unit (100) for determining the 3D orientation of the reference body based on the 2D image, for determining a plane having a specific orientation relative to the anatomical structure based on the 3D orientation of the reference body, with the reference body having a predetermined relation to the anatomical structure, and adapted to determine an angle between a viewing direction and said anatomical structure.

9. The device of claim 8, further comprising an input unit (300) for receiving an input representing an intended viewing direction and an output unit for providing data representing information for an adjustment of the C-arm based imaging device to achieve the intended viewing direction.

10. The device of claim 9, further comprising a monitor (400) for visualizing the information for an adjustment of the C-arm based imaging device (200).

11. A computer software product comprising computer software which when executed on the processing unit (100) of the device of claim 8, causes the device to perform the steps of the method of claim 1.

12. The computer software of claim 11, wherein the data provided by the output device are configured to automatically adjust the imaging direction of the C-arm based imaging device.

## Patentansprüche

1. Verfahren zum Unterstützen bei einer C-Arm Einstellung einer beabsichtigten Blickrichtung, wobei das Verfahren die Schritte umfasst:
Empfangen eines 2D Projektionsbildes einer anatomischen Struktur von einer C-Arm basierten Bildgebungsvorrichtung (200), wobei das 2D Projektionsbild eine Visualisierung eines Referenzkörpers (64) mit einer Struktur umfasst, die basierend auf dem 2D Projektionsbild eine Bestimmung einer 3D Orientierung des Referenzkörpers ermöglicht,
Bestimmen der 3D Orientierung des Referenzkörpers basierend auf dem 2D Projektionsbild,
Bestimmen einer Ebene mit einer spezifischen Orientierung relativ zu der anatomischen Struktur basierend auf der 3D Orientierung des Referenzkörpers, wobei der Referenzkörper eine vorbestimmte Beziehung zu der anatomischen Struktur aufweist,
Bestimmen eines Winkels zwischen der Blickrichtung und der anatomischen Struktur.

2. Verfahren nach Anspruch 1, wobei die Ebene mit der spezifischen Orientierung relativ zu der anatomischen Struktur eine Ebene ist, die mindestens parallel zu einer Ebene aus der Gruppe ist, die aus Sagittalebene, Frontalebene (FP) und Transversalebene (TP) besteht.

3. Verfahren nach Anspruch 1, wobei die Ebene mit der spezifischen Orientierung relativ zu der anatomischen Struktur ferner basierend auf mindestens einem anatomischen Merkmal der anatomischen Struktur, insbesondere mindestens einem Knochenmerkmal, bestimmt wird.

4. Verfahren nach Anspruch 3, wobei das Bestimmen der Ebene mit der spezifischen Orientierung relativ zu der anatomischen Struktur ferner auf 3D Daten basiert, die von einer Datenbank (600) empfangen werden, wobei die 3D Daten ein entsprechendes anatomisches Merkmal darstellen.

5. Verfahren nach Anspruch 1, wobei das 2D Projektionsbild ferner eine Visualisierung eines Implantats (20) umfasst und wobei die Ebene mit der spezifischen Orientierung relativ zu der anatomischen Struktur basierend auf einem Merkmal des Implantats bestimmt wird.

6. Verfahren nach Anspruch 5, wobei das Verfahren ferner den Schritt des Bestimmens einer Abweichung des visualisierten Merkmals des Implantats und einer erwarteten Visualisierung des Merkmals des Implantats umfasst.

7. Verfahren nach Anspruch 1, ferner umfassend die Schritte des Empfangens einer Eingabe, die eine beabsichtigte Blickrichtung darstellt, und des Bereitstellens von Informationen für eine Einstellung der C-Arm basierten Bildgebungsvorrichtung, um die beabsichtigte Blickrichtung zu erreichen.

8. Vorrichtung zum Unterstützen bei einer C-Arm Einstellung, wobei die Vorrichtung umfasst
einen Referenzkörper (64) mit einer Struktur, die basierend auf einem 2D Projektionsbild eine Bestimmung einer 3D Orientierung des Referenzkörpers ermöglicht,
eine Empfangseinheit zum Empfangen eines 2D Projektionsbildes einer anatomischen Struktur von einer C-Arm basierten Bildgebungsvorrichtung,
eine Verarbeitungseinheit (100) zum Bestimmen der 3D Orientierung des Referenzkörpers basierend auf dem 2D Bild, zum Bestimmen einer Ebene mit einer spezifischen Orientierung relativ zu der anatomischen Struktur basierend auf der 3D-Orientierung des Referenzkörpers, der eine vorbestimmte Beziehung zu der anatomischen Struktur aufweist, wobei die Verarbeitungseinheit zum Bestimmen eines Winkels zwischen einer Blickrichtung und der anatomischen Struktur angepasst ist.

9. Vorrichtung nach Anspruch 8, ferner umfassend eine Eingabeeinheit (300) zum Empfangen einer Eingabe, die eine beabsichtigte Blickrichtung darstellt, und eine Ausgabeeinheit zum Bereitstellen von Daten, die Informationen für eine Einstellung der C-Arm basierten Bildgebungsvorrichtung zum Erreichen der beabsichtigten Blickrichtung darstellen.

10. Vorrichtung nach Anspruch 9, ferner umfassend einen Monitor (400) zum Visualisieren der Informationen für eine Einstellung der C-Arm basierten Bildgebungsvorrichtung (200).

11. Computersoftwareprodukt umfassend Computersoftware, die, wenn sie auf der Verarbeitungseinheit (100) der Vorrichtung nach Anspruch 8 ausgeführt wird, bewirkt, dass die Vorrichtung die Verfahrensschritte nach Anspruch 1 ausführt.

12. Computersoftware nach Anspruch 11, wobei die von der Ausgabeeinheit bereitgestellten Daten zum automatischen Einstellen der Bildgebungsrichtung der C-Arm basierten Bildgebungsvorrichtung eingerichtet sind.

## Revendications

1. Un procédé d'assistance au réglage d'un bras en C selon une direction de visualisation voulue, le procédé comprenant les étapes consistant à :
recevoir une image de projection en 2D d'une structure anatomique d'un dispositif d'imagerie (200) basé sur le bras en C, l'image de projection en 2D comprenant une visualisation d'un corps de référence (64) ayant une structure permettant une détermination d'une orientation en 3D du corps de référence basée sur l'image de projection en 2D,
déterminer l'orientation en 3D du corps de référence sur la base de l'image de projection en 2D,
déterminer un plan ayant une orientation spécifique par rapport à la structure anatomique sur la base de l'orientation en 3D du corps de référence, le corps de référence ayant une relation prédéterminée avec la structure anatomique,
déterminer un angle entre la direction de visualisation et ladite structure anatomique.

2. Le procédé selon la revendication 1, dans lequel ledit plan ayant l'orientation spécifique par rapport à la structure anatomique est un plan qui est au moins parallèle à un plan situé hors du groupe constitué par un plan sagittal, un plan frontal (FP) et un plan transversal (TP).

3. Le procédé selon la revendication 1, dans lequel ledit plan ayant l'orientation spécifique par rapport à la structure anatomique est en outre déterminé sur la base d'au moins une caractéristique anatomique de la structure anatomique, en particulier sur au moins une caractéristique osseuse.

4. Le procédé selon la revendication 3, dans lequel le fait de déterminer ledit plan ayant l'orientation spécifique par rapport à la structure anatomique est en outre basé sur des données 3D reçues depuis une base de données (600), les données 3D représentant une caractéristique anatomique correspondante.

5. Le procédé selon la revendication 1, dans lequel l'image de projection en 2D comprend en outre une visualisation d'un implant (20) et dans lequel ledit plan ayant l'orientation spécifique par rapport à la structure anatomique est déterminé en fonction d'une caractéristique de l'implant.

6. Le procédé selon la revendication 5, le procédé comprenant en outre l'étape consistant à déterminer une déviation de la caractéristique visualisée de l'implant et une visualisation attendue de la caractéristique de l'implant.

7. Le procédé selon la revendication 1, comprenant en outre les étapes consistant à recevoir une entrée représentant une direction de visualisation voulue et à fournir des informations pour un réglage du dispositif d'imagerie basé sur le bras en C pour obtenir la direction de visualisation voulue.

8. Un dispositif d'aide au réglage d'un bras en C, le dispositif comprenant :
un corps de référence (64) ayant une structure permettant une détermination d'une orientation 3D du corps de référence à partir d'une image de projection en 2D,
une unité de réception pour recevoir une image de projection en 2D d'une structure anatomique à partir d'un dispositif d'imagerie basé sur le bras C,
une unité de traitement (100) pour déterminer l'orientation 3D du corps de référence sur la base de l'image en 2D, pour déterminer un plan ayant une orientation spécifique par rapport à la structure anatomique basée sur l'orientation 3D du corps de référence, le corps de référence ayant une relation prédéterminée à la structure anatomique, et adaptée pour déterminer un angle entre une direction de visualisation et ladite structure anatomique.

9. Le dispositif selon la revendication 8, comprenant en outre une unité d'entrée (300) pour recevoir une entrée représentant une direction de visualisation voulue et une unité de sortie pour fournir des données représentant des informations pour un réglage du dispositif d'imagerie basé sur le bras en C pour obtenir la direction de visualisation voulue.

10. Le dispositif selon la revendication 9, comprenant en outre un moniteur (400) pour visualiser les informations permettant un réglage du dispositif d'imagerie (200) basé sur le bras en C.

11. Un produit logiciel informatique comprenant un logiciel informatique qui, lorsqu'il est exécuté sur l'unité de traitement (100) du dispositif selon la revendication 8, amène le dispositif à mettre en oeuvre les étapes du procédé de la revendication 1.

12. Le logiciel d'ordinateur selon la revendication 11, dans lequel les données fournies par le dispositif de sortie sont configurées pour régler automatiquement la direction de formation d'image du dispositif d'imagerie basé sur le bras en C.
